# EUROPÄISCHE PATENTSCHRIFT

(11) **EP 3 015 082 B1**
(45) Veröffentlichungstag und Bekanntmachung des Hinweises auf die Patenterteilung: **11.07.2018**
(21) Anmeldenummer: 15187886.5
(22) Anmeldetag: 01.10.2015
(51) Int. Cl.: A61B 17/32, A61B 90/00, A61B 18/14

(54) **ZERLEGBARES MEDIZINISCHES INSTRUMENT**
DISMOUNTABLE MEDICAL INSTRUMENT
INSTRUMENT MEDICAL DEMONTABLE

(30) Priorität: 31.10.2014 DE 102014115873
(43) Veröffentlichungstag der Anmeldung: 04.05.2016
(73) Patentinhaber: Karl Storz SE & Co. KG, 78532 Tuttlingen (DE)
(72) Erfinder: Spycher, Raphael, 8264 Eschenz (CH); van Mil, Peter, 8404 Winterthur (CH)

(56) Entgegenhaltungen:
- EP-A1- 0 634 139
- EP-A1- 1 709 932
- WO-A1-2011/041520
- DE-A1- 3 227 417
- US-A- 4 600 384
- US-A- 5 873 886
- US-B1- 6 494 892

## Beschreibung

Die vorliegende Erfindung ist auf ein zerlegbares medizinisches Instrument, insbesondere auf ein zerlegbares medizinisches Instrument mit einem integrierten Fluidkanal, durch den beispielsweise abgetragenes Gewebe abgesaugt werden kann, bezogen.

Shaver und andere medizinische Instrumente zum mechanischen und/oder elektrochirurgischen Abtragen von Gewebe weisen oft einen integrierten Fluidkanal zum Spülen des Operationsorts oder zum Absaugen von abgetragenem Gewebe auf. Zur Steuerung des Fluidflusses und damit der Spül- oder Absaugwirkung kann ein Ventil vorgesehen sein. Insbesondere wenn durch den Fluidkanal abgetragenes Gewebe abgesaugt wird, ist eine gründliche mechanische Reinigung nach jeder Verwendung des medizinischen Instruments einschließlich des Fluidkanals erforderlich. Dies gilt besonders für ein im Fluidkanal vorgesehenes Ventil mit seinen vergleichsweise komplex geformten Oberflächen.

In EP 1 709 932 A1 ist eine Schnellkupplung zur Verbindung medizinischer Geräte beschrieben. Ein Kupplungselement 32, ist einerseits mittels einer ersten Bajonettkupplung 34, 35 mit einem Anschlussstück 2 verbunden und andererseits mittels einer zweiten Bajonettkupplung 41, 42, 51, 52 mit einem Handstück 1 verbindbar, um das Handstück 1 mit dem Anschlussstück 2 zu verbinden. Dabei verbleibt jedoch während der vorgesehenen Verwendung das Kupplungselement 32 fest mit dem Anschlussstück 2 verbunden.

In US 6,494,892 B1 ist ein chirurgisches Instrument mit einem angetriebenen Handstück 40 und einer als Einweg-Teil ausgestalteten chirurgischen Schneidvorrichtung 11 beschrieben. Zwischen dem Handstück 40 und der Schneidvorrichtung 11 können gleichzeitig zwei Renkverbindungen hergestellt werden, nämliche eine erste Renkverbindung zwischen einem Verbinder 60 und einem Verbindungsteil 18 und eine zweite Renkverbindung zwischen einem schneidenden Bauteil 35 und einem Antriebsverbinder.

In EP 0 634 139 A1 ist ein zerlegbares medizinisches Instrument ohne eine Renk- oder Bajonettverbindung beschrieben.

In DE 32 27 417 A1 (offenbart den Oberbegriff von Anspruch 1) ist eine Vorrichtung zum Befestigen einer Antriebseinrichtung im Körper eines zahnärztlichen Winkelstücks beschrieben. Ein hülsenartiges Gehäuse 5 weist an jedem Ende zwei Bajonettschlitze 12, 12' bzw. 13, 13' auf, mittels derer es einerseits mit einem Vorderteil 10 und andererseits mit einem Hinterteil 11 verbunden werden kann. Sowohl relativ zu dem Vorderteil 10 als auch relativ zu dem Hinterteil 11 sind jeweils lediglich zwei vorbestimmte Positionen vorgesehen, eine getrennte und eine verbundene. Die Verbindung des hülsenartigen Gehäuses 5 mit dem Vorderteil 10 ist nur von der Position des hülsenartigen Gehäuses 5 relativ zu dem Vorderteil 10 und nicht von der Position des hülsenartigen Gehäuses 5 relativ zu dem Hinterteil 11 abhängig. Die Verbindung des hülsenartigen Gehäuses 5 mit dem Hinterteil 11 ist nur von der Position des hülsenartigen Gehäuses 5 relativ zu dem Hinterteil 11 und nicht von der Position des hülsenartigen Gehäuses 5 relativ zu dem Vorderteil 10 abhängig.

In US 4,600,384 A ist eine Renkverbindung für ein beleuchtetes zahnmedizinisches Handstück beschrieben. Ein Verbindungsring 6 ist mittels eines Bajonettverschlusses 16 mit einem Trennring 18 verbunden. Der Trennring 18 ist rotierbar, jedoch nicht trennbar mit einem Handstückverbindungsring 24 verbunden. Eine zweite Renk- oder Bajonettverbindung ist nicht beschrieben.

Eine Aufgabe der vorliegenden Erfindung besteht darin, ein verbessertes zerlegbares medizinisches Instrument zu schaffen, das insbesondere auf möglichst einfache Weise möglichst weitgehend zerlegbar ist, um eine vollständige Reinigung zu vereinfachen.

Diese Aufgabe wird durch den Gegenstand des unabhängigen Anspruchs gelöst.

Weiterbildungen sind in den abhängigen Ansprüchen angegeben.

Ein zerlegbares medizinisches Instrument umfasst ein erstes Bauteil, ein zweites Bauteil, ein drittes Bauteil, eine erste Renkverbindungseinrichtung zum lösbaren mechanischen Verbinden des dritten Bauteils mit dem zweiten Bauteil durch eine erste Renkverbindung und eine zweite Renkverbindungseinrichtung zum lösbaren mechanischen Verbinden des dritten Bauteils mit dem ersten Bauteil durch eine zweite Renkverbindung, wobei bei einer ersten vorbestimmten Position des dritten Bauteils relativ zu dem zweiten Bauteil das dritte Bauteil nicht mit dem zweiten Bauteil verbunden ist, wobei bei einer zweiten vorbestimmten Position des dritten Bauteils relativ zu dem zweiten Bauteil das dritte Bauteil durch die erste Renkverbindung mit dem zweiten Bauteil mechanisch verbunden ist und das zweite Bauteil und das dritte Bauteil nicht mit dem ersten Bauteil verbunden sind, und wobei bei einer dritten vorbestimmten Position des dritten Bauteils relativ zu dem zweiten Bauteil und zu dem ersten Bauteil das dritte Bauteil durch die erste Renkverbindung mit dem zweiten Bauteil und durch die zweite Renkverbindung mit dem ersten Bauteil mechanisch starr verbunden ist.

Ein zerlegbares medizinisches Instrument umfasst ein erstes Bauteil, ein zweites Bauteil, ein drittes Bauteil, das mit dem zweiten Bauteil derart dauerhaft mechanisch verbunden ist, dass das dritte Bauteil relativ zu dem zweiten Bauteil rotierbar ist, und eine Renkverbindungseinrichtung zum lösbaren mechanischen Verbinden des dritten Bauteils mit dem ersten Bauteil durch eine Renkverbindung, wobei bei einer ersten vorbestimmten Position des dritten Bauteils relativ zu dem ersten Bauteil das dritte Bauteil nicht mit dem ersten Bauteil verbunden ist, und wobei bei einer zweiten vorbestimmten Position des dritten Bauteils relativ zu dem ersten Bauteil das dritte Bauteil durch die Renkverbindung mit dem ersten Bauteil mechanisch starr verbunden ist.

Das dritte Bauteil ist mit dem zweiten Bauteil insbesondere derart dauerhaft mechanisch verbunden, dass das dritte Bauteil relativ zu dem zweiten Bauteil rotierbar ist. Das erste Bauteil und das zweite Bauteil sind insbesondere so ausgebildet, dass es nur eine vorbestimmte Position des zweiten Bauteils relativ zu dem ersten Bauteil gibt, in der das dritte Bauteil die erste vorbestimmte Position und die zweite vorbestimmte Position relativ zu dem ersten Bauteil erreichen kann. In diesem Fall sind die erste vorbestimmte Position des dritten Bauteils relativ zu dem ersten Bauteil auch eine erste vorbestimmte Position des dritten Bauteils relativ zu dem zweiten Bauteil und die zweite vorbestimmte Position des dritten Bauteils relativ zu dem ersten Bauteil auch eine zweite vorbestimmte Position des dritten Bauteils relativ zu dem zweiten Bauteil. Die Renkverbindung und die Renkverbindungseinrichtung des zuletzt genannten zerlegbaren medizinischen Instruments entsprechen der zweiten Renkverbindung bzw. der zweiten Renkverbindungseinrichtung der nachfolgend dargestellten Ausführungsformen und Varianten.

Das zerlegbare medizinische Instrument ist insbesondere ein Shaver oder ein anderes medizinisches Instrument zum Abtragen von Gewebe auf mechanischem und/oder elektrochirurgischem Weg. Das erste Bauteil umfasst insbesondere einen Handgriff oder eine Handhabe zum manuellen Halten, Führen und Bewegen des zerlegbaren medizinischen Instruments während seines vorgesehenen Einsatzes bzw. seiner vorgesehenen Verwendung im Rahmen einer medizinischen Maßnahme. Das medizinische Instrument ist vorgesehen und ausgebildet, um nach jeder Verwendung weitgehend zerlegt zu werden, wobei insbesondere das erste Bauteil, das zweite Bauteil und das dritte Bauteil zerstörungsfrei voneinander getrennt werden können. Im zerlegten Zustand kann das medizinische Instrument weitgehend oder vollständig gereinigt werden. Dazu trägt bei, dass Oberflächen, die bei der zusammengesetzten Konfiguration zwischen dem ersten Bauteil und dem zweiten Bauteil oder zwischen dem zweiten Bauteil und dem dritten Bauteil oder zwischen dem ersten Bauteil und dem dritten Bauteil liegen, im zerlegten Zustand offen liegen. Dabei liegen insbesondere auch Oberflächen von Fluidkanälen oder anderen Hohlräumen des medizinischen Instruments offen und sind einer unmittelbaren manuellen oder maschinellen Reinigung zugänglich.

Eine Renkverbindung (oft auch als Bajonettverbindung oder Bajonettkupplung bezeichnet) ist eine formschlüssige und in der Regel lösbare ohne Verwendung von Werkzeug zerstörungsfrei lösbare mechanische Verbindung zwischen zwei Bauteilen. Eine Renkverbindung wird hergestellt, indem die Bauteile zunächst durch eine Translationsbewegung (in der Regel eine reine Translationsbewegung ohne damit einhergehender Rotationsbewegung) aneinander angenähert und danach relativ zueinander rotiert werden. Durch einen umgekehrten Bewegungsablauf - zunächst Rotation, dann Translation - können die beiden Bauteile wieder voneinander getrennt werden.

Die erste Renkverbindungseinrichtung umfasst insbesondere eine oder mehrere Knaggen, Stege oder Nuten an einem der beiden beteiligten Bauteile, die ausgebildet, angeordnet und vorgesehen sind, um Knaggen oder Stege am anderen beteiligten Bauteil zu hintergreifen oder in Nuten am anderen beteiligten Bauteil einzugreifen. Insbesondere weist jeweils eines der beiden beteiligten Bauteile nach radial innen ragende Knaggen oder Stege auf, die hinter korrespondierende, nach radial außen ragende Knaggen oder Stege oder in eine oder mehrere nach radial außen offene Nuten am anderen beteiligten Bauteil eingreifen können.

Nach der Reinigung des medizinischen Instruments können das erste Bauteil, das zweite Bauteil und das dritte Bauteil in einer vorgesehenen Weise zusammengesetzt werden, um die vorgesehene Funktionalität des medizinischen Instruments herzustellen. Dies ist nachfolgend für das medizinische Instrument beschrieben, bei dem das zweite Bauteil und das dritte Bauteil nicht dauerhaft, sondern durch eine erste Renkverbindung miteinander verbunden sind. Bein dem medizinischen Instrument, bei dem das zweite Bauteil und das dritte Bauteil dauerhaft mechanische verbunden sind, entfallen Schritte zur Herstellung der ersten Renkverbindung zwischen dem zweiten Bauteil und dem dritten Bauteil.

Zum Zusammensetzen des medizinischen Instruments ist es insbesondere vorgesehen, zunächst das dritte Bauteil in einer ersten vorbestimmten Position an das zweite Bauteil anzusetzen. Die erste vorbestimmte Position umfasst eine erste vorbestimmte Winkelposition bzw. räumliche Orientierung des dritten Bauteils relativ zu dem zweiten Bauteil, in der das dritte Bauteil durch eine erste, insbesondere geradlinige reine Translationsbewegung (ohne damit einhergehende Rotationsbewegung) an das zweite Bauteil angenähert wird bis das dritte Bauteil die erste vorbestimmte Position relativ zu dem zweiten Bauteil erreicht hat.

Ausgehend von der ersten vorbestimmten Position kann das dritte Bauteil durch eine erste reine Rotationsbewegung um eine vorbestimmte Rotationsachse in die zweite vorbestimmte Position relativ zu dem zweiten Bauteil gebracht werden. In der zweiten vorbestimmten Position des dritten Bauteils relativ zu dem zweiten Bauteil sind das dritte Bauteil und das zweite Bauteil insofern mechanisch miteinander verbunden, als sie nicht mehr durch eine rein translatorische Bewegung voneinander trennbar sind. Die Rotationsachse der ersten Rotationsbewegung ist insbesondere parallel zu der Richtung der vorangehenden ersten Translationsbewegung. Mit der ersten Translationsbewegung und der nachfolgenden ersten Rotationsbewegung wird die erste Renkverbindung zwischen dem dritten Bauteil und dem zweiten Bauteil hergestellt.

Wenn das dritte Bauteil durch die erste Renkverbindung mit dem zweiten Bauteil mechanisch verbunden ist, können das zweite Bauteil und das dritte Bauteil als Baugruppe gemeinsam an das erste Bauteil herangeführt werden. Dies erfolgt insbesondere in einer zweiten geradlinigen reinen Translationsbewegung in einer Richtung parallel zur Rotationsachse der ersten Renkverbindung. Bei dieser reinen Translationsbewegung erreicht das zweite Bauteil relativ zu dem ersten Bauteil insbesondere bereits seine endgültige und für die vorgesehene Verwendung des medizinischen Instruments vorbestimmte Position relativ zu dem ersten Bauteil.

Mit einer nachfolgenden zweiten Rotationsbewegung des dritten Bauteils relativ zu dem zweiten Bauteil und zu dem ersten Bauteil wird die zweite Renkverbindung zwischen dem dritten Bauteil und dem ersten Bauteil hergestellt. Die Rotation erfolgt insbesondere um die Rotationsachse der ersten Renkverbindung. Mit dieser zweiten Rotationsbewegung erreicht das dritte Bauteil die dritte vorbestimmte Position relativ zu dem zweiten Bauteil und zu dem ersten Bauteil, in der es durch die erste Renkverbindung mit dem zweiten Bauteil und durch die zweite Renkverbindung mit dem ersten Bauteil mechanisch verbunden ist.

Die Ausbildung des zerlegbaren medizinischen Instruments mit drei Bauteilen, die durch zwei Renkverbindungen miteinander verbindbar sind, kann eine sehr weitgehende Zerlegung und damit eine weitgehende Offenlegung innerer Oberflächen des medizinischen Instruments und damit eine einfache und vollständige Reinigung ermöglichen. Die lösbare mechanische Verbindung der Bauteile durch Renkverbindungen kann eine einfache und schnelle Zerlegung und nachfolgende Zusammensetzung des medizinischen Instruments auch durch ungeübtes Personal ermöglichen.

Die erste Renkverbindung zwischen dem dritten Bauteil und dem zweiten Bauteil verhindert, dass gleichzeitig drei nicht miteinander verbundene Bauteile gehandhabt werden müssen. Vielmehr müssen jederzeit höchstens zwei Bauteile oder in sich zusammenhängende Baugruppen gehalten und relativ zueinander bewegt werden, nämlich zunächst das zweite Bauteil und das dritte Bauteil und danach das erste Bauteil und die Baugruppe aus dem zweiten Bauteil und dem dritten Bauteil.

Durch die indirekte mechanische Verbindung zwischen dem ersten Bauteil und dem zweiten Bauteil über das dritte Bauteil müssen das erste Bauteil und das zweite Bauteil zu keinem Zeitpunkt relativ zueinander rotiert werden. Dies schafft zusätzliche konstruktive Freiheitsgrade, beispielsweise kann das zweite Bauteil in einer Weise in das erste Bauteil eingreifen, die eine Rotation des zweiten Bauteils relativ zu dem ersten Bauteil nicht zulässt und damit eine unmittelbare Renkverbindung zwischen dem ersten Bauteil und dem zweiten Bauteil verhindert würde.

Bei einem zerlegbaren medizinischen Instrument, wie es hier beschrieben ist, ist am in der vorgesehenen Weise funktionsfähig zusammengesetzten medizinischen Instrument insbesondere das zweite Bauteil zwischen dem ersten Bauteil und dem dritten Bauteil angeordnet.

Anders ausgedrückt ist das dritte Bauteil insbesondere an einer vom ersten Bauteil teilweise abgewandten Seite des zweiten Bauteils angeordnet. Mit einer Anordnung des zweiten Bauteils zwischen dem ersten Bauteil und dem dritten Bauteil bzw. einer Anordnung des dritten Bauteils an einer vom ersten Bauteil abgewandten Seite des zweiten Bauteil ist eine unmittelbare Berührung zwischen dem ersten Bauteil und dem dritten Bauteil nicht ausgeschlossen, wie sie tatsächlich im Bereich der zweiten Renkverbindungseinrichtung erfolgt.

Die Anordnung des zweiten Bauteils zwischen dem ersten Bauteil und dem dritten Bauteil schafft insbesondere eine formschlüssige Definition des Orts des zweiten Bauteils und kann eine Bewegung des zweiten Bauteils relativ zu dem ersten Bauteil und zu dem dritten Bauteil weitgehend oder vollständig unterbinden.

Ein zerlegbares medizinisches Instrument, wie es hier beschrieben ist, umfasst insbesondere ferner eine Rasteinrichtung zum Halten des dritten Bauteils zumindest entweder in der zweiten vorbestimmten Position oder in der dritten vorbestimmten Position relativ zu dem zweiten Bauteil.

Die Rasteinrichtung umfasst insbesondere eine Ausnehmung oder Vertiefung und eine gegen die Rückstellkraft einer elastischen Einrichtung bewegbare und zur Ausnehmung oder Vertiefung korrespondierende vorstehende Einrichtung. Insbesondere sind die Ausnehmung oder Vertiefung am dritten Bauteil und die elastische Einrichtung und die vorstehende Einrichtung am zweiten Bauteil angeordnet. Alternativ sind die Ausnehmung oder Vertiefung am zweiten Bauteil und die elastische Einrichtung und die vorstehende Einrichtung am dritten Bauteil angeordnet. Die elastische Einrichtung umfasst beispielsweise eine Spiralfeder in einer Bohrung, wobei die vorstehende Einrichtung eine Kugel umfasst, die durch Formschluss an einem vollständigen Verlassen der Bohrung gehindert und durch die Feder in eine Position, in der sie teilweise aus der Bohrung hervorragt, gedrückt wird.

Die Rasteinrichtung vereinfacht das Zusammensetzen des zerlegbaren medizinischen Instruments insbesondere, indem sie das dritte Bauteil sowohl in der zweiten vorbestimmten Position als auch in der dritten vorbestimmten Position formschlüssig hält und ein unbeabsichtigtes Lösen der ersten Renkverbindung oder der zweiten Renkverbindung verhindert. Ferner schafft die Rasteinrichtung eine taktile und/oder hörbare Rückmeldung bei Erreichen der zweiten oder der dritten vorbestimmten Position, die das Zusammensetzen ebenfalls vereinfacht.

Ein zerlegbares medizinisches Instrument, wie es hier beschrieben ist, umfasst insbesondere ferner eine Einrichtung zur Unterbindung einer Rotation des zweiten Bauteils relativ zu dem ersten Bauteil, wenn das dritte Bauteil sich in der dritten vorbestimmten Position befindet.

Bei einem zerlegbaren medizinischen Instrument, wie es hier beschrieben ist, umfasst die Einrichtung zur Unterbindung einer Rotation insbesondere einen Rohrstutzen am zweiten Bauteil und eine korrespondierende Öffnung am ersten Bauteil oder einen Rohrstutzen am ersten Bauteil und eine korrespondierende Öffnung am zweiten Bauteil.

Alternativ umfasst die Einrichtung zur Unterbindung einer Rotation beispielsweise einen Stift oder eine Nase oder einen anderen konvexen Bereich am zweiten Bauteil, der bzw. die in eine korrespondierende Öffnung am ersten Bauteil eingreift oder einen Stift oder eine Nase oder einen anderen konvexen Bereich am ersten Bauteil, der bzw. die in eine korrespondierende Öffnung am zweiten Bauteil eingreift.

Eine Einrichtung zur Unterbindung einer Rotation des zweiten Bauteils relativ zu dem ersten Bauteil kann eine vorbestimmte Orientierung des zweiten Bauteils relativ zu dem ersten Bauteil bewirken und ein unbeabsichtigtes Lösen der zweiten Renkverbindung zwischen dem dritten Bauteil und dem ersten Bauteil verhindern. Ein Rohrstutzen und eine korrespondierende Öffnung können beispielsweise in einer Richtung parallel zur Rotationsachse der Renkverbindungen ineinander eingreifen, wobei der Rohrstutzen und die Öffnung nicht rotationssymmetrisch zur Rotationsachse sind. Insbesondere sind der Rohrstutzen und die Öffnung von der Rotationsachse der Renkverbindungen beabstandet.

Ein Rohrstutzen und eine korrespondierende Öffnung können insbesondere eine fluidische Verbindung zwischen dem ersten Bauteil und dem zweiten Bauteil schaffen.

Bei einem zerlegbaren medizinischen Instrument, wie es hier beschrieben ist, umfassen das erste Bauteil und das zweite Bauteil jeweils insbesondere einen oder mehrere Abschnitte eines Fluidkanals zum Transport eines Fluids, wobei die Abschnitte des Fluidkanals im zerlegten Zustand des medizinischen Instruments für eine Reinigung zugänglich sind.

Insbesondere umfassen das erste Bauteil und das zweite Bauteil jeweils Teile eines Fluidkanals zum Transport eines aus einem Operationsgebiet abgesaugten Fluids, das beispielsweise abgetragenes Gewebe enthalten kann.

Insbesondere bilden der oben erwähnte Rohrstutzen und die oben erwähnte Öffnung, in die der Rohrstutzen eingreifen kann, Teile eines Fluidkanals in dem medizinischen Instrument.

Bei einem zerlegbaren medizinischen Instrument, wie es hier beschrieben ist, sind insbesondere das medizinische Instrument zum Abtragen von Gewebe und der Fluidkanal zum Absaugen von abgetragenem Gewebe vorgesehen und ausgebildet.

Das zerlegbare medizinische Instrument ist insbesondere ein Shaver mit einer Klinge oder einer Drahtschlaufe, an die eine hochfrequente Hochspannung angelegt werden kann, um Gewebe mechanisch und/oder elektrochirurgisch abzutragen. Die Klinge oder die Drahtschlaufe können durch einen in oder an der medizinischen Vorrichtung vorgesehenen Motor rotierbar sein.

Ein zerlegbares medizinisches Instrument, wie es hier beschrieben ist, umfasst insbesondere ferner ein Ventil zum teilweisen oder vollständigen Unterbrechen des Fluidkanals im zweiten Bauteil.

Das Ventil kann zum An- und Ausschalten, Verändern, Steuern oder Regeln der Absaugleistung oder - im Falle eines ein Spül-Fluid transportierenden Fluidkanals - der Spülleistung vorgesehen und ausgebildet sein. Durch die Zerlegbarkeit des medizinischen Instruments, insbesondere durch die Trennbarkeit des zweiten Bauteils vom ersten Bauteil können innere Oberflächen des Ventils freigelegt und einer mechanischen Reinigung zugänglich gemacht werden.

Bei einem zerlegbaren medizinischen Instrument, wie es hier beschrieben ist, weist das erste Bauteil insbesondere einen vorstehenden Bereich auf, wobei das zweite Bauteil im Wesentlichen ringartig ist, und wobei am in der vorgesehenen Weise funktionsfähig zusammengesetzten medizinischen Instrument das zweite Bauteil den vorstehenden Bereich am ersten Bauteil umschließt.

Bei einem zerlegbaren medizinischen Instrument, wie es hier beschrieben ist, ist das dritte Bauteil insbesondere im Wesentlichen ringartig, wobei am in der vorgesehenen Weise funktionsfähig zusammengesetzten medizinischen Instrument das dritte Bauteil den vorstehenden Bereich am ersten Bauteil umschließt.

Ein Bauteil ist ringartig, wenn es in mathematischem Sinne mehrfach (insbesondere zweifach) zusammenhängend ist bzw. die Topologie eines Kreisrings aufweist. Das Bauteil muss dabei weder kreisringförmig sein noch beispielsweise die Gestalt eines Torus aufweisen. Ein ringartiges Bauteil weist jedoch eine das Bauteil vollständig durchdringende Öffnung auf, in die der vorstehende Bereich am ersten Bauteil eingeführt werden kann.

Bei einem zerlegbaren medizinischen Instrument, wie es hier beschrieben ist, umschließen das zweite Bauteil und das dritte Bauteil in ihrer durch die erste Renkverbindung verbundenen Konfiguration insbesondere einen im Wesentlichen kreiszylindrischen Innenraum.

Der vorstehende Bereich am ersten Bauteil weist insbesondere eine zu dem vom zweiten Bauteil und vom dritten Bauteil umschlossenen Innenraum korrespondierende Gestalt auf.

Die Rotationsachse der Renkverbindungen und die Translationsrichtungen der Renkverbindungen sind insbesondere parallel zu oder identisch mit einer Längsachse oder einer Symmetrieachse des vorstehenden Bereichs am ersten Bauteil.

Bei einem zerlegbaren medizinischen Instrument, wie es hier beschrieben ist, sind insbesondere in dem vorstehenden Bereich ein erster Abschnitt des Fluidkanals im Wesentlichen parallel zur Längsachse des vorstehenden Bereichs und ein zweiter Abschnitt des Fluidkanals im Wesentlichen orthogonal zur Längsachse des vorstehenden Bereichs angeordnet.

Der vorstehende Bereich am ersten Bauteil enthält also insbesondere einen L-förmigen oder T-förmigen Teil des Fluidkanals.

Ein zerlegbares medizinisches Instrument, wie es hier beschrieben ist, umfasst insbesondere ferner zwei jeweils ringartig den vorstehenden Bereich am ersten Bauteil umschließende Dichtungen, wobei der zweite Abschnitt des Fluidkanals in einer Öffnung zwischen den Dichtungen endet.

Die den vorstehenden Bereich am ersten Bauteil jeweils ringartig umschließenden Dichtungen sind beispielsweise O-Ringe in entsprechenden ringförmigen, nach außen und/oder in axialer Richtung sich öffnenden kreisförmigen Nuten. Die ringartigen Dichtungen können durch Vulkanisation oder auf andere Weise mit dem ersten Bauteil stoffschlüssig verbunden sein.

Bei einem zerlegbaren medizinischen Instrument, wie es hier beschrieben ist, ist das zweite Bauteil insbesondere ausgebildet, um am in der vorgesehenen Weise funktionsfähig zusammengesetzten medizinischen Instrument an beiden Dichtungen dichtend anzuliegen.

Bei einem zerlegbaren medizinischen Instrument, wie es hier beschrieben ist, weist der zweite Abschnitt des Fluidkanals insbesondere an zwei voneinander abgewandten Seiten des vorstehenden Bereichs am ersten Bauteil jeweils eine Öffnung auf.

Die durch die beiden Öffnungen gebildeten Zweige des Fluidkanals können durch einen ringförmigen Hohlraum zwischen dem ersten Bauteil und dem zweiten Bauteil und zwischen den beiden Dichtungen wieder zusammengeführt werden. Die beiden Öffnungen können eine Reinigung des Fluidkanals deutlich vereinfachen, indem beispielsweise eine Bürste oder ein reinigender Fluidstrom quer durch den vorstehenden Bereich von einer der beiden Öffnungen zur anderen hin geführt wird.

Bei einem Verfahren zum Zusammensetzen eines zerlegbaren medizinischen Instruments werden ein drittes Bauteil und ein zweites Bauteil durch eine erste Renkverbindung miteinander verbunden und dann das dritte Bauteil durch eine zweite Renkverbindung mit einem ersten Bauteil verbunden.

Das Verfahren ist insbesondere anwendbar auf oder ausführbar mit einem zerlegbaren medizinischen Instrument, wie es hier beschrieben ist.

### Kurzbeschreibung der Figuren

Nachfolgend werden Ausführungsformen anhand der beigefügten Figuren näher erläutert. Es zeigen:
- Figur 1: eine schematische axonometrische Darstellung eines zerlegbaren medizinischen Instruments;
- Figur 2: eine weitere schematische axonometrische Darstellung des zerlegbaren medizinischen Instruments aus Figur 1;
- Figur 3: eine schematische Darstellung eines dritten Bauteils des zerlegbaren medizinischen Instruments aus den Figuren 1 und 2;
- Figur 4: eine weitere schematische axonometrische Darstellung des zerlegbaren medizinischen Instruments aus den Figuren 1 bis 3;
- Figur 5: eine weitere schematische axonometrische Darstellung des zerlegbaren medizinischen Instruments aus den Figuren 1 bis 4;
- Figur 6: eine weitere schematische axonometrische Darstellung des zerlegbaren medizinischen Instruments aus den Figuren 1 bis 5;
- Figur 7: eine weitere schematische axonometrische Darstellung des zerlegbaren medizinischen Instruments aus den Figuren 1 bis 6.

### Beschreibung der Ausführungsformen

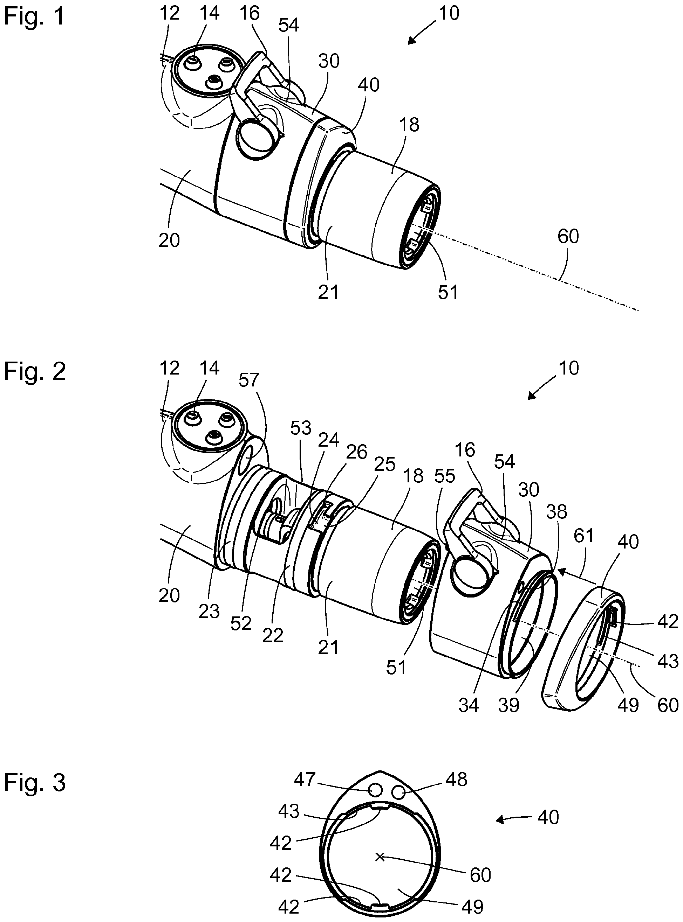

Figur 1 zeigt eine schematische axonometrische Darstellung eines zerlegbaren medizinischen Instruments oder eines Teils eines zerlegbaren medizinischen Instruments 10 mit einem Handgriff 12 in einem proximalen Bereich des zerlegbaren medizinischen Instruments 10. Nahe dem Handgriff 12 sind eine oder mehrere Tasten, Druckknöpfe, Schalter oder andere Bedienelemente 14 sowie ein Ventilbetätigungshebel 16 angeordnet. Das zerlegbare medizinische Instrument 10 ist insbesondere ein Shaver zum mechanischen und/oder elektrochirurgischen Abtragen von Gewebe. Die Bedienelemente 14 sind beispielsweise zum Auswählen eines Betriebsmodus, zum Ein- und Ausschalten einer Hochspannung und/oder eines motorischen Antriebs eines oszillierenden oder rotierenden Werkzeugs oder zum Verändern der Frequenz, der Spannung oder der Drehzahl vorgesehen.

Der dargestellte Teil des zerlegbaren medizinischen Instruments 10 umfasst an seinem distalen Ende (in Figur 1: rechts) einen im Wesentlichen kreiszylindrischen Kupplungsbereich zur mechanischen Kopplung mit einem Werkzeug oder mit einem proximalen Ende eines Schafts, dessen distales Ende ein Werkzeug aufweist oder mit einem Werkzeug verbindbar ist. Das zerlegbare medizinische Instrument 10 weist im Kupplungsbereich 18 einen ersten Abschnitt 51 eines Fluidkanals zum Absaugen von abgetragenem Gewebe auf. Der erste Abschnitt 51 des Fluidkanals erstreckt sich in axialer Richtung des im Wesentlichen kreiszylindrischen Kupplungsbereichs 18. In Figur 1 ist lediglich der Eingang des ersten, axialen Abschnitts 51 des Fluidkanals erkennbar.

Der in Figur 1 dargestellte Teil des zerlegbaren medizinischen Instruments 10 weist drei Bauteile 20, 30, 40 auf. Am ersten Bauteil 20 ist ein vorstehender Bereich 21 vorgesehen, der den Kupplungsbereich 18 umfasst. Das zweite Bauteil 30 und das dritte Bauteil 40 sind jeweils ringartig ausgebildet und umschließen jeweils den vorstehenden Bereich 21.

Der in Figur 1 dargestellte Teil des medizinischen Instruments 10 ist in die Bauteile 20, 30, 40 zerstörungsfrei und ohne Verwendung von Werkzeug zerlegbar und kann aus den Bauteilen 20, 30, 40 ohne Verwendung von Werkzeug einfach und schnell zusammengesetzt werden. Das Zusammensetzen der Bauteile 20, 30, 40 ist anhand der Figuren 2 bis 5 dargestellt.

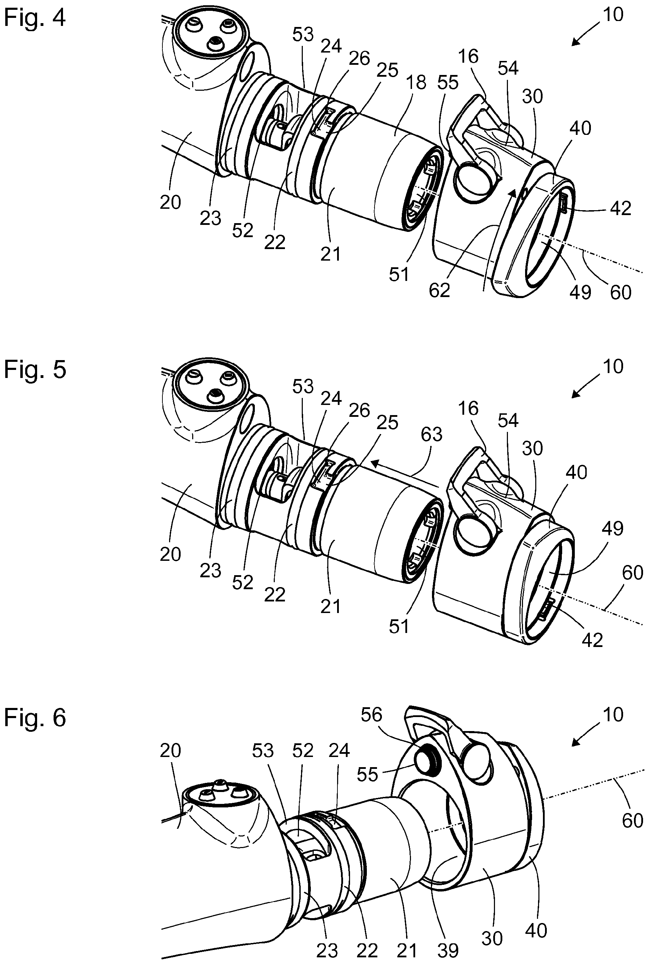

Figur 2 zeigt eine weitere schematische axonometrische Darstellung der Bauteile 20, 30, 40 des zerlegbaren medizinischen Instruments 10 aus Figur 1. In Figur 2 sind die Bauteile 20, 30, 40 voneinander beabstandet, jedoch in der relativen Orientierung, in der sie - wie anhand der Figuren 4 bis 7 dargestellt - zusammengesetzt werden können, gezeigt.

Der vorstehende Bereich 21 am ersten Bauteil 20 weist - insbesondere von nachfolgend beschriebenen Merkmalen abgesehen - im Wesentlichen die Gestalt eines Kreiszylinders mit einer Symmetrieachse 60 auf. Der vorstehende Bereich 21 am ersten Bauteil 20 weist proximal des Kupplungsbereichs 18 bzw. zwischen dem Handgriff 12 und dem Kupplungsbereich 18 zwei Dichtungen 22, 23 auf, die den vorstehenden Bereich 21 jeweils ringförmig, insbesondere kreisringförmig umgeben. Die Dichtungen 22, 23 sind beispielsweise O-Ringe in entsprechenden in axialer Richtung oder - wie beim dargestellten Beispiel - nach radial außen sich öffnenden Nuten. Jede der beiden Dichtungen kann alternativ durch Vulkanisieren mit dem ersten Bauteil 20 stoffschlüssig verbunden sein.

Der vorstehende Bereich 21 des ersten Bauteils 20 weist ferner eine oder mehrere jeweils L-förmige Nuten 24 auf, von denen in Figur 2 eine sichtbar und dargestellt ist. Die L-förmige Nut 24 umfasst einen axialen Abschnitt 25 parallel zur Symmetrieachse 60 des vorstehenden Bereichs 21 und einen in Richtung des Umfangs des vorstehenden Bereichs 21 sich erstreckenden Abschnitt 26.

Das zweite Bauteil 30 ist im Wesentlichen ringförmig und umschließt einen im Wesentlichen kreiszylindrischen Innenraum 39, dessen Querschnitt im Wesentlichen dem Querschnitt des vorstehenden Bereichs 21 am ersten Bauteil 20 entspricht. Das zweite Bauteil 30 weist an seinem distalen Rand einen oder mehrere jeweils kreisbogenförmige und nach radial außen ragende Stege 34 auf, von denen in Figur 2 einer sichtbar und dargestellt ist. Ferner weist das zweite Bauteil 30 an seiner distalen und dem dritten Bauteil 40 zugewandten Seite eine Rasteinrichtung 38 auf. Die Rasteinrichtung 38 umfasst beispielsweise eine Kugel in einer Bohrung, die in der Bohrung parallel zur Symmetriesachse 60 bewegbar, jedoch durch Formschluss an einem vollständigen Verlassen der Bohrung gehindert ist und durch eine Feder in eine Position geschoben wird, in der die Kugel teilweise aus der Bohrung hervorragt.

Das dritte Bauteil 40 umfasst einen oder mehrere kreisbogenförmige und nach radial innen ragende Stege 43, von denen in Figur 2 nur einer sichtbar und dargestellt ist. Die kreisbogenförmigen Stege 34 am zweiten Bauteil 30 und die kreisbogenförmigen Stege 43 am dritten Bauteil 40 sind derart korrespondierend ausgebildet und angeordnet, dass die Stege 34, 43 einander hintergreifend eine formschlüssige Verbindung zwischen dem zweiten Bauteil 30 und dem dritten Bauteil 40 bilden können. Insbesondere sind sowohl am zweiten Bauteil 30 als auch am dritten Bauteil 40 jeweils zwei Stege einander gegenüberliegend angeordnet, die jeweils sich über etwas weniger als 90 Grad erstrecken. Die Stege 34 am zweiten Bauteil 30 und die korrespondierenden Stege 43 am dritten Bauteil 40 bilden somit zusammen eine erste Renkverbindungseinrichtung zur Bildung einer ersten Renkverbindung zwischen dem zweiten Bauteil 30 und dem dritten Bauteil 40.

Am dritten Bauteil 40 sind ferner eine oder mehrere jeweils nach radial innen ragende Knaggen oder Nasen 42 angeordnet, von denen in Figur 2 nur eine sichtbar und dargestellt ist. Die Knaggen 42 am dritten Bauteil 40 korrespondieren hinsichtlich ihrer Gestalt und ihrer Anordnung zu den L-förmigen Nuten 24 am ersten Bauteil 20. Durch eine Translationsbewegung und eine nachfolgende Rotationsbewegung des dritten Bauteils 40 relativ zu dem ersten Bauteil 20 kann jede Knagge 42 zunächst durch den axialen Abschnitt 25 und dann in den in Richtung des Umfangs sich erstreckenden Abschnitt 26 der korrespondierenden L-förmigen Nut 24 eingeführt werden. Die Nuten 24 am ersten Bauteil 20 und die korrespondierenden Knaggen 42 am dritten Bauteil 40 bilden somit eine zweite Renkverbindungseinrichtung zur formschlüssigen mechanischen Verbindung des dritten Bauteils 40 mit dem ersten Bauteil 20 durch eine zweite Renkverbindung.

Wie bereits erwähnt ist im vorstehenden Bereich 21 am ersten Bauteil 20 ein erster, axialer Abschnitt 51 eines Fluidkanals vorgesehen, der sich parallel oder im Wesentlichen parallel zur Symmetrieachse 60 des vorstehenden Bereichs 21 am ersten Bauteil 20 über die gesamte Länge oder einen großen Teil der Länge des vorstehenden Bereichs 21 am ersten Bauteil 20 erstreckt. Der erste, axiale Abschnitt 51 des Fluidkanals weist insbesondere einen kreisförmigen oder im Wesentlichen kreisförmigen Querschnitt auf.

Ein zweiter, radialer Abschnitt 52 des Fluidkanals erstreckt sich orthogonal zur Symmetrieachse 60 des vorstehenden Bereichs 21 am ersten Bauteil 20 vom ersten Abschnitt 51 bis zu einer Öffnung 53 zwischen den Dichtungen 22, 23 am vorstehenden Bereich 21 am ersten Bauteil 20. Bei dem dargestellten Beispiel sind zwei einander gegenüberliegende zweite, radiale Abschnitte 52 des Fluidkanals vorgesehen, die in zwei einander gegenüberliegenden Öffnungen 53 am vorstehenden Bereich 21 des ersten Bauteils 20 enden. Die beiden zweiten, radialen Abschnitte 52 des Fluidkanals sind insbesondere durch eine Durchgangsbohrung oder ein Durchgangsloch, das sich orthogonal zur Symmetrieachse 60 des vorstehenden Bereichs 21 am ersten Bauteil 20 erstreckt, gebildet. Insgesamt weist der Fluidkanal 51, 52 innerhalb des vorstehenden Bereichs 21 am ersten Bauteil 20 somit eine T-förmige Gestalt auf.

Im zweiten Bauteil 30 ist ein Ventil 54 vorgesehen, das durch Schwenken des Ventilbetätigungshebels 16 um eine Schwenkachse orthogonal zur Symmetrieachse 60 des vorstehenden Bereichs 21 am ersten Bauteil 20 geöffnet und geschlossen werden kann. Ferner ist an einer dem ersten Bauteil 20 zugewandten Seite des zweiten Bauteils 30 ein Rohrstutzen 55 vorgesehen, der in Figur 2 fast vollständig verdeckt ist. Der Rohrstutzen 55 erstreckt sich parallel oder im Wesentlichen parallel zur Symmetrieachse 60 des vorstehenden Bereichs 21 am ersten Bauteil 20. Im zweiten Bauteil 30 ist ein im Wesentlichen L-förmiger Abschnitt des Fluidkanals angeordnet, der in Figur 2 nicht sichtbar ist, in dem das Ventil 54 und der Rohrstützen 55 liegen.

Am ersten Bauteil 20 ist an einer dem zweiten Bauteil 30 zugewandten Stirnfläche eine zu dem Rohrstutzen 55 am zweiten Bauteil 30 korrespondierende Öffnung 57 vorgesehen. In der vorgesehenen Weise am funktionsfähig zusammengesetzten medizinischen Instrument 10 (vgl. Figur 1) greift der Rohrstutzen 55 am zweiten Bauteil 30 in die Öffnung 57 am ersten Bauteil 20 ein, und der erwähnte L-förmige Abschnitt im zweiten Bauteil 30 verbindet den zweiten, radialen Abschnitt 52 des Fluidkanals mit der Öffnung 57 im ersten Bauteil 20. Das zusammengesetzte medizinische Instrument 10 weist somit einen Fluidkanal auf, dessen Bestandteile der erste, axiale Abschnitt 51, der oder die zweiten, radialen Abschnitte 52, das Ventil 54, der Rohrstutzen 55 und die Öffnung 57 im ersten Bauteil 20 sind.

Mittels des Ventils 54 im zweiten Bauteil 30 kann der Fluidkanal verschlossen bzw. unterbrochen und geöffnet bzw. durchgängig gemacht werden. Wenn die Öffnung 57 im ersten Bauteil 20 beispielsweise mit einer Saug- oder Vakuumpumpe gekoppelt ist, kann mittels des Ventilbetätigungshebels 16 und des Ventils 54 eine Absaugwirkung am medizinischen Instrument 10 an- und ausgeschaltet werden. Optional kann das Ventil 54 so ausgestaltet sein, dass die Absaugwirkung stufenlos oder in Stufen einstellbar ist. Wenn die Öffnung 57 im ersten Bauteil 20 über eine Förderpumpe mit einem Spülfluid-Reservoir gekoppelt ist, kann mittels des Ventilbetätigungshebels 16 und des Ventils 54 ein Spülfluid-Fluss am zerlegbaren medizinischen Instrument 10 an- und ausgeschaltet und optional stufenlos oder in Stufen eingestellt werden.

Figur 3 zeigt eine schematische Darstellung des dritten Bauteils 40 des anhand der Figuren 1 und 2 dargestellten medizinischen Instruments 10. In Figur 3 ist die dem zweiten Bauteil 30 zugewandte Stirnfläche des dritten Bauteils 40 sichtbar.

Das ringartige bzw. im Wesentlichen ringförmige dritte Bauteil 40 umschließt einen im Wesentlichen kreisförmigen oder kreiszylindrischen Innenraum 49, dessen Querschnitt im Wesentlichen dem Querschnitt des vorstehenden Bereichs 21 am ersten Bauteil 20 entspricht. Zwei Knaggen 42 am 40 ragen in den Innenraum 40. Zwei einander gegenüberliegende, jeweils kreisbogenförmige und nach innen ragende Stege 43 nehmen jeweils einen Winkel von fast 90 Grad ein und sind durch zwei Lücken, die sich jeweils über etwas mehr als 90 Grad erstrecken, getrennt. In der in Figur 3 gezeigten, dem zweiten Bauteil 30 (vgl. Figur 2) zuzuwendenden Stirnfläche sind eine erste Ausnehmung 47 und eine zweite Ausnehmung 48 vorgesehen, die hinsichtlich ihrer Gestalt und ihrer Anordnung zu der Rasteinrichtung 38 am zweiten Bauteil 30 (vgl. Figur 2) korrespondieren.

Figur 4 zeigt eine weitere schematische axonometrische Darstellung der Bauteile 20, 30, 40 aus den Figuren 1 bis 3. Die Art der Darstellung entspricht derjenigen der Figuren 1 und 2.

Die in Figur 4 gezeigte Konfiguration unterscheidet sich von der in Figur 2 gezeigten dadurch, dass das dritte Bauteil 40 durch eine erste reine Translationsbewegung parallel zur Symmetrieachse 60 relativ zu dem zweiten Bauteil 30 in eine erste vorbestimmte Position relativ zu dem zweiten Bauteil 30 gebracht ist. Die erste Translationsbewegung ist in Figur 2 durch einen Pfeil 61 angedeutet.

In der in Figur 4 dargestellten ersten vorbestimmten Position des dritten Bauteils 40 relativ zu dem zweiten Bauteil 30 sind das zweite Bauteil 30 und das dritte Bauteil 40 noch nicht miteinander formschlüssig mechanisch verbunden, sondern können durch eine reine Translationsbewegung wieder voneinander getrennt werden.

Ausgehend von der in Figur 4 dargestellten ersten vorbestimmten Position des dritten Bauteils 40 relativ zu dem zweiten Bauteil 30 kann das dritte Bauteil 40 durch eine durch einen Pfeil 62 angedeutete erste Rotationsbewegung um die Symmetrieachse 60 in eine in Figur 5 gezeigte zweite vorbestimmte Position relativ zu dem zweiten Bauteil 30 gebracht werden.

Figur 5 zeigt eine weitere schematische axonometrische Darstellung des zerlegbaren medizinischen Instruments 10 aus den Figuren 1 bis 4. Die Art der Darstellung entspricht derjenigen der Figuren 1, 2 und 4.

In Figur 5 ist die bereits erwähnte zweite vorbestimmte Position des dritten Bauteils 40 relativ zu dem zweiten Bauteil 30 gezeigt. In dieser zweiten vorbestimmten Position hintergreifen die nach radial innen ragenden Stege 43 am dritten Bauteil 40 die nach radial außen ragenden Stege 34 am zweiten Bauteil 30 (vgl. Figuren 2 und 3) teilweise. Dadurch ist eine formschlüssige mechanische Verbindung, nämlich eine erste Renkverbindung, zwischen dem zweiten Bauteil 30 und dem dritten Bauteil 40 hergestellt, die nicht durch eine rein translatorische Relativbewegung wieder getrennt werden kann. Dadurch bilden das zweite Bauteil 30 und das dritte Bauteil 40 eine Baugruppe.

Bei der in Figur 5 gezeigten zweiten vorbestimmten Position des dritten Bauteils 40 relativ zu dem zweiten Bauteil 30 greift die Rasteinrichtung 38 am zweiten Bauteil 30 (vgl. Figuren 2 und 4) in die erste Ausnehmung 47 am dritten Bauteil 40 ein (vgl. Figur 3). Dadurch wird das dritte Bauteil 40 elastisch-formschlüssig in der zweiten vorbestimmten Position relativ zu dem zweiten Bauteil 30 gehalten.

Figur 6 zeigt eine weitere schematische axonometrische Darstellung des ersten Bauteils 20 und der aus dem zweiten Bauteil 30 und dem dritten Bauteil 40 gebildeten Baugruppe aus einer etwas anderen Perspektive bzw. Blickrichtung.

In Figur 6 ist der Rohrstutzen 55 an der dem ersten Bauteil 20 zugewandten Stirnfläche des zweiten Bauteils 30 sichtbar. Am äußeren Umfang des Rohrstutzens 55 ist eine Dichtung 56 aus einem Elastomer oder einem anderen elastischen Material vorgesehen, die beispielsweise durch Vulkanisation stoffschlüssig mit dem zweiten Bauteil 30 verbunden ist. Dabei bildet die Dichtung 56 fast die gesamte äußere Oberfläche des Rohrstutzens 55 oder zumindest einen Teil davon.

Die gesamte äußere Oberfläche oder der durch die Dichtung 56 gebildete Teil der äußeren Oberfläche des Rohrstutzens 55 ist beispielsweise konusförmig bzw. weist die Gestalt eines Ausschnitts einer Mantelfläche eines Kreiskegels auf. Alternativ kann die äußere Kontur des Querschnitts der Dichtung 56 oder des gesamten Rohrstutzens beispielsweise Stufen aufweisen oder treppenförmig ausgebildet sein. In diesem Fall kann beim in der vorgesehenen Weise funktionsfähig zusammengesetzten medizinischen Instrument 10 (vgl. Figur 1) die Dichtung 56 in mehreren schmalen ringförmigen Bereichen am Rand der Öffnung 57 (vgl. Figuren 2, 4, 5) anliegen. Dadurch kann die Dichtwirkung der Dichtung 56 verbessert werden.

Die Dichtung 56 kann neben ihrer Dichtwirkung optional einen Toleranzausgleich zwischen dem ersten Bauteil 20 und dem zweiten Bauteil 30 bewirken. Das erste Bauteil 20 und das zweite Bauteil 30 sind insbesondere so ausgebildet, dass sie ein vorbestimmtes Spiel aufweisen, um reibungsarm zusammengesetzt werden zu können. Dieses Spiel kann durch die Elastizität der Dichtung 56 teilweise oder vollständig aufgehoben werden.

Ausgehend von der in den Figuren 5 und 6 gezeigten Konfiguration kann die Baugruppe aus dem zweiten Bauteil 30 und dem dritten Bauteil 40 durch eine zweite reine Translationsbewegung, die in Figur 5 durch einen Pfeil 63 angedeutet ist, an das erste Bauteil 20 angesetzt werden.

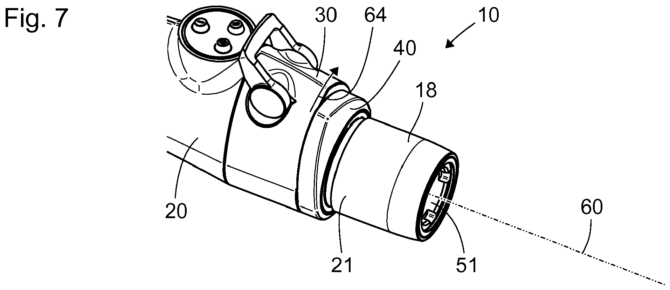

Figur 7 zeigt eine weitere schematische axonometrische Darstellung der Bauteile 20, 30, 40 aus den Figuren 1 bis 6. Die Art der Darstellung entspricht derjenigen der Figuren 1, 2, 4 und 5.

In Figur 7 ist die Konfiguration gezeigt, die ausgehend von der in den Figuren 5 und 6 gezeigten Konfiguration durch die durch den Pfeil 63 in Figur 5 angedeutete zweite Translationsbewegung der Baugruppe aus dem zweiten Bauteil 30 und dem dritten Bauteil 40 relativ zu dem ersten Bauteil 20 erreicht wird. Bei dieser zweiten Translationsbewegung werden die Knaggen 42 am dritten Bauteil 40 (vgl. Figuren 2 bis 5) in den axialen Abschnitten 25 der L-förmigen Nuten 24 bewegt.

In der in Figur 7 dargestellten Konfiguration greift der Rohrstutzen 55 am zweiten Bauteil 30 (vgl. Figur 6) in die Öffnung 57 am ersten Bauteil 20 (vgl. Figuren 2, 4 und 5) ein, und die Dichtung 56 am zweiten Bauteil 30 liegt in einem oder mehreren ringförmigen Bereichen am Rand der Öffnung 57 am ersten Bauteil 20 dichtend an. Ferner liegt bei der in Figur 7 gezeigten Konfiguration die Wandfläche des im Wesentlichen kreiszylindrischen Innenraums 39 des zweiten Bauteils 30 (vgl. insbesondere Figuren 2 und 6) an den Dichtungen 22, 23 am vorstehenden Bereich 21 am ersten Bauteil 20 (vgl. Figuren 2, 4, 5 und 6) dichtend an. Damit ist der erwähnte Fluidkanal im Inneren des medizinischen Instruments 10 vollständig hergestellt und gegenüber der Außenwelt abgedichtet.

Bei der in Figur 7 gezeigten Konfiguration kann die Baugruppe aus dem zweiten Bauteil 30 und dem dritten Bauteil 40 jedoch durch eine einfache Translationsbewegung parallel zur Symmetrieachse 60 des vorstehenden Bereichs 21 an dem ersten Bauteil 20 vom ersten Bauteil 20 getrennt werden. Dabei würden die Knaggen 42 am dritten Bauteil 40 (vgl. Figuren 2 bis 5) lediglich in den axialen Abschnitten 25 der L-förmigen Nuten 24 bewegt.

Ausgehend von der in Figur 7 gezeigten Konfiguration kann das dritte Bauteil 40 durch eine durch einen Pfeil 64 angedeutete zweite Rotationsbewegung 64 relativ zu dem ersten Bauteil 20 und zu dem zweiten Bauteil 30 mit dem ersten Bauteil 20 verbunden werden. Dabei werden die Knaggen 42 am dritten Bauteil 40 (vgl. Figuren 2 bis 5) in die in Richtung des Umfangs des vorstehenden Bereichs 21 des ersten Bauteils 20 sich erstreckenden Abschnitte 26 der L-förmigen Nuten 24 hinein bewegt, und das dritte Bauteil 40 erreicht die in Figur 1 gezeigte dritte vorbestimmte Position relativ zu dem ersten Bauteil 20 und zu dem zweiten Bauteil 30.

Bei der in Figur 1 gezeigten dritten vorbestimmten Position des dritten Bauteils 40 relativ zu dem zweiten Bauteil 30 greift die Rasteinrichtung 38 am zweiten Bauteil 30 (vgl. Figuren 2 und 4) in die zweite Ausnehmung 48 am dritten Bauteil 40 (vgl. Figur 3) ein. Dadurch wird das dritte Bauteil 40 rastend bzw. elastisch-formschlüssig in der in Figur 1 gezeigten dritten vorbestimmten Position relativ zu dem zweiten Bauteil 30 (und indirekt auch relativ zu dem ersten Bauteil 20) gehalten.

In dieser dritten vorbestimmten Position ist durch den Eingriff der Knaggen 42 am dritten Bauteil 40 (vgl. Figuren 2 bis 5) in die in Richtung des Umfangs des vorstehenden Bereichs 21 des ersten Bauteils 20 sich erstreckenden Abschnitte 26 der L-förmigen Nuten 24 eine formschlüssige Verbindung zwischen dem dritten Bauteil 40 und dem ersten Bauteil 20 geschaffen. Diese formschlüssige Verbindung kann nicht durch eine einfache Translationsbewegung getrennt werden. Die L-förmigen Nuten 24 am vorstehenden Bereich 21 am ersten Bauteil 20 und die korrespondierenden Knaggen 42 am dritten Bauteil 40 bilden eine zweite Renkverbindungseinrichtung zur formschlüssigen und zerstörungsfrei lösbaren mechanischen Verbindung des ersten Bauteils 20 mit dem dritten Bauteil 40 durch eine zweite Renkverbindung.

Durch die erste Renkverbindung zwischen dem zweiten Bauteil 30 und dem dritten Bauteil 40 und die zweite Renkverbindung zwischen dem dritten Bauteil 40 und dem ersten Bauteil 20 sind mittelbar auch das erste Bauteil 20 und das dritte Bauteil 40 miteinander mechanisch starr verbunden. Zusätzlich unterbinden die Anordnung des zweiten Bauteils 30 zwischen dem ersten Bauteil 20 und dem dritten Bauteil 40 eine Translationsbewegung und der Formschluss zwischen dem Rohrstutzen 55 am zweiten Bauteil 30 und der Öffnung 57 im ersten Bauteil 20 eine Rotationsbewegung des zweiten Bauteils 30 relativ zu dem ersten Bauteil 20.

### Bezugszeichen

- 10: zerlegbares medizinisches Instrument oder Teil davon
- 12: Handgriff in einem proximalen Bereich des zerlegbaren medizinischen Instruments 10
- 14: Bedienelement nahe dem Handgriff 12
- 16: Ventilbetätigungshebel nahe dem Handgriff 12
- 18: im Wesentlichen zylindrischer Kupplungsbereich für Schaft oder Werkzeug
- 20: erstes Bauteil des zerlegbaren medizinischen Instruments 10
- 21: vorstehender Bereich am ersten Bauteil 20
- 22: erste Dichtung am vorstehenden Bereich 21 am ersten Bauteil 20
- 23: zweite Dichtung am vorstehenden Bereich 21 am ersten Bauteil 20
- 24: L-förmige Nut am vorstehenden Bereich 21 am ersten Bauteil 20
- 25: axialer Abschnitt der L-förmigen Nut 24
- 26: in Richtung des Umfangs sich erstreckender Abschnitt der L-förmigen Nut 24
- 30: zweites Bauteil des zerlegbaren medizinischen Instruments 10
- 34: kreisbogenförmiger und nach radial außen ragender Steg am zweiten Bauteil 30
- 38: Rasteinrichtung am zweiten Bauteil 30
- 39: im Wesentlichen kreiszylindrischer Innenraum des zweiten Bauteils 30
- 40: drittes Bauteil des zerlegbaren medizinischen Instruments 10
- 42: zur L-förmigen Nut 24 am ersten Bauteil 20 korrespondierende Knagge am dritten Bauteil 40
- 43: zum Steg 34 am zweiten Bauteil 30 korrespondierender Steg am dritten Bauteil 40
- 47: erste zur Rasteinrichtung 38 am zweiten Bauteil 30 korrespondierende Ausnehmung am dritten Bauteil 40
- 48: zweite zur Rasteinrichtung 38 am zweiten Bauteil 30 korrespondierende Ausnehmung am dritten Bauteil 40
- 49: im Wesentlichen kreiszylindrischer Innenraum des dritten Bauteils 40
- 51: erster, axialer Abschnitt eines Fluidkanals im zerlegbaren medizinischen Instrument 10
- 52: zweiter, radialer Abschnitt eines Fluidkanals im zerlegbaren medizinischen Instrument 10
- 53: Öffnung am vorstehenden Bereich 21 des ersten Bauteils 20 und radial äußeres Ende des zweiten, radialen Abschnitts des Fluidkanals 52
- 54: Ventil im zweiten Bauteil 30
- 55: Rohrstutzen am zweiten Bauteil 30
- 56: Dichtung am Rohrstutzen 55
- 57: zum Rohrstutzen 55 am zweiten Bauteil 30 korrespondierende Öffnung am ersten Bauteil 20
- 60: Symmetrieachse des vorstehenden Bereichs 21 am ersten Bauteil 20 und gleichzeitig Rotationsachse der Renkverbindungseinrichtung 34, 43 und der Renkverbindung 24, 42
- 61: geradlinige Translationsbewegung des dritten Bauteils 40 zu dem zweiten Bauteil 30 hin
- 62: erste Rotationsbewegung des dritten Bauteils 40 relativ zu dem zweiten Bauteil 30
- 63: geradlinige Translationsbewegung der Baugruppe aus dem zweiten Bauteil 30 und dem dritten Bauteil 40 zu dem ersten Bauteil 20 hin.
- 64: zweite Rotationsbewegung des dritten Bauteils 40 relativ zu dem zweiten Bauteil 30 und zu dem ersten Bauteil 20

## Patentansprüche

1. **Zerlegbares medizinisches Instrument** (10) mit:
einem **ersten Bauteil** (20);
einem **zweiten Bauteil** (30);
einem **dritten Bauteil** (40);
einer **ersten Renkverbindungseinrichtung** (34, 43) zum lösbaren mechanischen Verbinden des dritten Bauteils (40) mit dem zweiten Bauteil (30) durch eine erste Renkverbindung,
einer **zweiten Renkverbindungseinrichtung** (24, 42) zum lösbaren mechanischen Verbinden des dritten Bauteils (40) mit dem ersten Bauteil (20) durch eine zweite Renkverbindung;
bei einer **ersten vorbestimmten Position** des dritten Bauteils (40) relativ zu dem zweiten Bauteil (30) das dritte Bauteil (40) nicht mit dem zweiten Bauteil (30) verbunden ist,
bei einer **zweiten vorbestimmten Position** des dritten Bauteils (40) relativ zu dem zweiten Bauteil (30) das dritte Bauteil (40) durch die erste Renkverbindung mit dem zweiten Bauteil (30) mechanisch verbunden ist und das zweite Bauteil (30) und das dritte Bauteil (40) nicht mit dem ersten Bauteil (20) verbunden sind,
**dadurch gekennzeichnet, dass** bei einer **dritten vorbestimmten Position** des dritten Bauteils (40) relativ zu dem zweiten Bauteil (30) und zu dem ersten Bauteil (20) das dritte Bauteil (40) durch die erste Renkverbindung mit dem zweiten Bauteil (30) und durch die zweite Renkverbindung mit dem ersten Bauteil (20) mechanisch starr verbunden ist,
bei dem das erste Bauteil (20) einen **vorstehenden Bereich** (21) aufweist,das zweite Bauteil (30) im wesentlichen **ringartig** ist,
am in der vorgesehenen Weise funktionsfähig zusammengesetzten medizinischen Instrument (10) das zweite Bauteil (30) den vorstehenden Bereich (21) am ersten Bauteil (20) umschließt.

2. Zerlegbares medizinisches Instrument (10) nach einem der vorangehenden Ansprüche, wobei am in der vorgesehenen Weise funktionsfähig zusammengesetzten medizinischen Instrument (10) das **zweite Bauteil** (30) **zwischen** dem ersten Bauteil (20) und dem dritten Bauteil (40) angeordnet ist.

3. Zerlegbares medizinisches Instrument (10) nach einem der vorangehenden Ansprüche, ferner mit:
einer **Rasteinrichtung** (38, 47, 48) zum Halten des dritten Bauteils (40) zumindest entweder in der zweiten vorbestimmten Position oder in der dritten vorbestimmten Position relativ zu dem zweiten Bauteil (30).

4. Zerlegbares medizinisches Instrument (10) nach einem der vorangehenden Ansprüche, ferner mit:
einer **Einrichtung** (55, 57) **zur Unterbindung einer Rotation** des zweiten Bauteils (30) relativ zu dem ersten Bauteil (20), wenn das dritte Bauteil (40) sich in der dritten vorbestimmten Position befindet.

5. Zerlegbares medizinisches Instrument (10) nach dem vorangehenden Anspruch, bei dem die Einrichtung zur Unterbindung einer Rotation einen **Rohrstutzen** (55) am zweiten Bauteil (30) und eine korrespondierende **Öffnung** (57) am ersten Bauteil (20) oder einen Rohrstutzen am ersten Bauteil (20) und eine korrespondierende Öffnung am zweiten Bauteil (30) umfasst.

6. Zerlegbares medizinisches Instrument (10) nach einem der vorangehenden Ansprüche, bei dem das erste Bauteil (20) und das zweite Bauteil (30) jeweils einen oder mehrere Abschnitte (51, 52, 53, 54, 55, 57) eines **Fluidkanals** zum Transport eines Fluids umfassen, wobei die Abschnitte (51, 52, 53, 54, 55, 57) des Fluidkanals im zerlegten Zustand des medizinischen Instruments (10) für eine Reinigung zugänglich sind.

7. Zerlegbares medizinisches Instrument (10) nach Anspruch 6, ferner mit:
einem **Ventil** (54) zum teilweisen oder vollständigen Unterbrechen des Fluidkanals (51, 52, 53,54,55,57) im zweiten Bauteil (30).

8. Zerlegbares medizinisches Instrument (10) nach dem vorangehenden Anspruch, bei dem
das dritte Bauteil (40) im Wesentlichen **ringartig** ist,
am in der vorgesehenen Weise funktionsfähig zusammengesetzten medizinischen Instrument (10) das dritte Bauteil (40) den vorstehenden Bereich (21) am ersten Bauteil (20) umschließt.

9. Zerlegbares medizinisches Instrument (10) nach Anspruch 8 in Rückbezug auf Anspruch 6, bei dem in dem vorstehenden Bereich (21) am ersten Bauteil (20) ein erster Abschnitt (51) des Fluidkanals im Wesentlichen parallel zur Längsachse (60) des vorstehenden Bereichs (21) und ein zweiter Abschnitt (52) des Fluidkanals im Wesentlichen orthogonal zur Längsachse (60) angeordnet sind.

10. Zerlegbares medizinisches Instrument (10) nach dem vorangehenden Anspruch, ferner mit:
zwei jeweils ringartig den vorstehenden Bereich (21) am ersten Bauteil (20) umschließenden Dichtungen (22, 23), wobei der zweite Abschnitt (52) des Fluidkanals in einer Öffnung (53) zwischen den Dichtungen (22, 23) endet.

11. Zerlegbares medizinisches Instrument (10) nach dem vorangehenden Anspruch, bei dem der zweite Abschnitt (52) des Fluidkanals an zwei von einander abgewandten Seiten des vorstehenden Bereichs (21) am ersten Bauteil (20) jeweils eine Öffnung (53) aufweist.

## Claims

1. Dismantlable medical instrument (10) with:
a first component (20);
a second component (30);
a third component (40);
a first bayonet connection mechanism (34, 43) for releasable mechanical connection of the third component (40) to the second component (30) by a first bayonet connection,
a second bayonet connection mechanism (24, 42) for releasable mechanical connection of the third component (40) to the first component (20) by a second bayonet connection;
wherein, in a first predetermined position of the third component (40) relative to the second component (30), the third component (40) is not connected to the second component (30),
wherein, in a second predetermined position of the third component (40) relative to the second component (30), the third component (40) is mechanically connected to the second component (30) by the first bayonet connection, and the second component (30) and the third component (40) are not connected to the first component (20),
**characterized in that**, in a third predetermined position of the third component (40) relative to the second component (30) and to the first component (20), the third component (40) is mechanically rigidly connected to the second component (30) by the first bayonet connection and to the first component (20) by the second bayonet connection,
the first component (20) having a protruding region (21), and the second component (30) being substantially ring-like,
the second component (30) enclosing the protruding region (21) on the first component (20) when the medical instrument (10) is assembled in the intended manner ready for operation.

2. Dismantlable medical instrument (10) according to one of the preceding claims, wherein the second component (30) is arranged between the first component (20) and the third component (40) when the medical instrument (10) is assembled in the intended manner ready for operation.

3. Dismantlable medical instrument (10) according to one of the preceding claims, also with:
a detent mechanism (38, 47, 48) for holding the third component (40) at least either in the second predetermined position or in the third predetermined position relative to the second component (30).

4. Dismantlable medical instrument (10) according to one of the preceding claims, also with:
a mechanism (55, 57) for suppressing a rotation of the second component (30) relative to the first component (20) when the third component (40) is located in the third predetermined position.

5. Dismantlable medical instrument (10) according to the preceding claim, in which the mechanism for suppressing a rotation comprises a tubular stub (55) on the second component (30) and a corresponding opening (57) on the first component (20), or a tubular stub on the first component (20) and a corresponding opening on the second component (30).

6. Dismantlable medical instrument (10) according to one of the preceding claims, in which the first component (20) and the second component (30) each comprise one or more portions (51, 52, 53, 54, 55, 57) of a fluid channel for transporting a fluid, wherein the portions (51, 52, 53, 54, 55, 57) of the fluid channel are accessible for cleaning in the dismantled state of the medical instrument (10) .

7. Dismantlable medical instrument (10) according to Claim 6, also with:
a valve (54) for partially or completely interrupting the fluid channel (51, 52, 53, 54, 55, 57) in the second component (30).

8. Dismantlable medical instrument (10) according to the preceding claim, in which
the third component (40) is substantially ring-like,
the third component (40) encloses the protruding region (21) on the first component (20) when the medical instrument (10) is assembled in the intended manner ready for operation.

9. Dismantlable medical instrument (10) according to Claim 8 with back-reference to Claim 6, in which, in the protruding region (21) on the first component (20), a first portion (51) of the fluid channel is arranged substantially parallel to the longitudinal axis (60) of the protruding region (21), and a second portion (52) of the fluid channel is arranged substantially orthogonal to the longitudinal axis (60).

10. Dismantlable medical instrument (10) according to the preceding claim, also with:
two seals (22, 23) which each annularly enclose the protruding region (21) on the first component (20), wherein the second portion (52) of the fluid channel ends in an opening (53) between the seals (22, 23).

11. Dismantlable medical instrument (10) according to the preceding claim, in which the second portion (52) of the fluid channel has an opening (53) on each of two opposite sides of the protruding region (21) on the first component (20).

## Revendications

1. Instrument médical démontable (10), comprenant :
un premier composant (20) ;
un deuxième composant (30) ;
un troisième composant (40) ;
un premier dispositif de liaison à baïonnette (34, 43) pour la connexion mécanique amovible du troisième composant (40) au deuxième composant (30) par le biais d'une première liaison à baïonnette,
un deuxième dispositif de liaison à baïonnette (24, 42) pour la connexion mécanique amovible du troisième composant (40) au premier composant (20) par le biais d'une deuxième liaison à baïonnette ; le troisième composant (40), dans une première position prédéterminée du troisième composant (40) par rapport au deuxième composant (30), n'étant pas connecté au deuxième composant (30),
le troisième composant (40), dans une deuxième position prédéterminée du troisième composant (40) par rapport au deuxième composant (30), étant connecté mécaniquement par la première liaison à baïonnette au deuxième composant (30) et le deuxième composant (30) et le troisième composant (40) n'étant pas connectés au premier composant (20),
**caractérisé en ce que** le troisième composant (40), dans une troisième position prédéterminée du troisième composant (40) par rapport au deuxième composant (30) et par rapport au premier composant (20), est connecté mécaniquement rigidement par la première liaison à baïonnette au deuxième composant (30) et par la deuxième liaison à baïonnette au premier composant (20),
le premier composant (20) présentant une région saillante (21), le deuxième composant (30) étant sensiblement annulaire,
le deuxième composant (30), au niveau de l'instrument médical (10) assemblé fonctionnellement de la manière prévue, entourant la région saillante (21) sur le premier composant (20) .

2. Instrument médical démontable (10) selon l'une quelconque des revendications précédentes, dans lequel le deuxième composant (30) est disposé entre le premier composant (20) et le troisième composant (40) au niveau de l'instrument médical (10) assemblé fonctionnellement de la manière prévue.

3. Instrument médical démontable (10) selon l'une quelconque des revendications précédentes, comprenant en outre :
un dispositif d'encliquetage (38, 47, 48) pour retenir le troisième composant (40) au moins soit dans la deuxième position prédéterminée soit dans la troisième position prédéterminée par rapport au deuxième composant (30).

4. Instrument médical démontable (10) selon l'une quelconque des revendications précédentes, comprenant en outre :
un dispositif (55, 57) pour supprimer une rotation du deuxième composant (30) par rapport au premier composant (20) lorsque le troisième composant (40) se trouve dans la troisième position prédéterminée.

5. Instrument médical démontable (10) selon la revendication précédente, dans lequel le dispositif pour supprimer une rotation comprend une tubulure (55) au niveau du deuxième composant (30) et une ouverture correspondante (57) au niveau du premier composant (20) ou une tubulure au niveau du premier composant (20) et une ouverture correspondante au niveau du deuxième composant (30).

6. Instrument médical démontable (10) selon l'une quelconque des revendications précédentes, dans lequel le premier composant (20) et le deuxième composant (30) comprennent chacun une ou plusieurs portions (51, 52, 53, 54, 55, 57) d'un canal de fluide pour le transport d'un fluide, les portions (51, 52, 53, 54, 55, 57) du canal de fluide, dans l'état démonté de l'instrument médical (10) étant accessibles en vue d'un nettoyage.

7. Instrument médical démontable (10) selon la revendication 6, comprenant en outre :
une soupape (54) pour interrompre partiellement ou complètement le canal de fluide (51, 52, 53, 54, 55, 57) dans le deuxième composant (30).

8. Instrument médical démontable (10) selon la revendication précédente, dans lequel
le troisième composant (40) est essentiellement annulaire,
le troisième composant (40) entoure la région saillante (21) sur le premier composant (20) au niveau de l'instrument médical (10) assemblé fonctionnellement de la manière prévue.

9. Instrument médical démontable (10) selon la revendication 8 lorsqu'elle se rapporte à la revendication 6, dans lequel, dans la région saillante (21) sur le premier composant (20), une première portion (51) du canal de fluide est disposée essentiellement parallèlement à l'axe longitudinal (60) de la région saillante (21) et une deuxième portion (52) du canal de fluide est disposée essentiellement perpendiculairement à l'axe longitudinal (60).

10. Instrument médical démontable (10) selon la revendication précédente, comprenant en outre : deux joints d'étanchéité (22, 23) entourant chacun de manière annulaire la région saillante (21) sur le premier composant (20), la deuxième portion (52) du canal de fluide se terminant dans une ouverture (53) entre les joints d'étanchéité (22, 23) .

11. Instrument médical démontable (10) selon la revendication précédente, dans lequel la deuxième portion (52) du canal de fluide présente à chaque fois une ouverture (53) au niveau de deux côtés mutuellement opposés de la région saillante (21) sur le premier composant (20).
